(19) Europäisches Patentamt
European Patent Office
Office européen des brevets

(11) Veröffentlichungsnummer: **0 003 540**
**B1**

(12) **EUROPÄISCHE PATENTSCHRIFT**

(21) Anmeldenummer: **79100241.3**

(22) Anmeldetag: **29.01.79**

(51) Int. Cl.³: **C 07 D 271/10,**
C 07 D 285/12,
C 07 D 249/08

# (54) N-Azolylalkyl-aniline sowie Verfahren zu ihrer Herstellung

(30) Priorität: **10.02.78 DE 2805756**

(43) Veröffentlichungstag der Anmeldung:
**22.08.79 Patentblatt 79/17**

(45) Bekanntmachung des Hinweises auf die Patenterteilung:
**01.10.80 Patentblatt 80/20**

(84) Benannte Vertragsstaaten:
**BE CH DE FR GB IT NL**

(56) Entgegenhaltungen:
**DE - A - 2 145 513**
**DE - A - 2 625 285**
**GB - A - 1 307 283**

(73) Patentinhaber: **BAYER AG**
**Zentralbereich Patente, Marken und Lizenzen**
**D - 5090 Leverkusen 1, Bayerwerk (DE)**

(72) Erfinder: **Stetter, Jörg, Dr.**
**Pahlkestrasse 3**
**D - 5600 Wuppertal 1 (DE)**
**Draber, Wilfried, Dr.**
**In den Birken 81**
**D - 5600 Wuppertal 1 (DE)**
**Thomas, Rudolf, Dr.**
**Wilkhausstrasse 129**
**D - 5600 Wuppertal 2 (DE)**
**Lunkenheimer, Winfried, Dr.**
**Bismarckstrasse 29**
**D - 5600 Wuppertal (DE)**

**EP 0 003 540 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## N-Azolylalkyl-aniline sowie Verfahren zu ihrer Herstellung

Die vorliegende Anmeldung betrifft neue N-Azolylalkylaniline, mehrere Verfahren zu ihrer Herstellung sowie ihre Verwendung als Zwischenprodukte zur Synthese von N-Azolylalkylhalogenacetaniliden, welche herbizide Eigenschaften besitzen.

N-Alkyl-N-heteroaryl-aniline und deren Verwendung als Zwischenprodukte zur Synthese von pharmakologisch wirksamen Stoffen sind bereits bekannt geworden (vgl. DE-OS 26 25 285). Entsprechende Verbindungen, in denen das Stickstoffatom mit einem substituierten Phenylrest und einer Azolylalkyl-Gruppe verbunden ist, werden jedoch nicht erwähnt.

Weiterhin sind Benzylthiol-Derivate von verschiedenen Azolen bekannt (vgl. GB-PS 1 307 283). Analoge N-Azolylalkyl-aniline werden allerdings nicht offenbart.

Ferner sind 5-(ω-Amino-alkyl)-ox-2,4-diazole und deren Verwendung als Zwischenprodukte zur Herstellung von Fungiziden bekannt (vgl. DE-OS 21 45 513). Entsprechende Stoffe, die sich von 1,2-Diazolen ableiten, werden hingegen nicht beschrieben.

Schließlich ist bereits bekannt geworden, daß man 2,6-Diäthyl-N-methoxymethyl-chloracetanilid zur selektiven Unkrautbekämpfung verwenden kann (vgl. R. Wegler, Chemie der Pflanzenschutz- und Schädlingsbekämpfungsmittel, Band 5, Seite 255, Springer-Verlag (1977). Diese Verbindung ist jedoch, vor allem gegenüber Dicotylen nicht immer ausreichend wirksam und in ihrer Selektivität nicht immer ganz befriedigend.

Es wurden nun als neue Verbindungen die N-Azolylalkylaniline der Formel

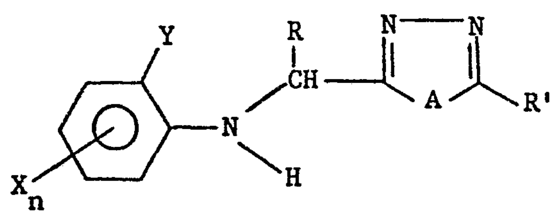

(I)

in welcher

A für Sauerstoff, Schwefel oder die Gruppierung $>NR^2$ steht,

R für Wasserstoff oder Alkyl steht,

$R^1$ für Wasserstoff, Alkyl, Halogenalkyl, Alkenyl, Alkinyl, Cycloalkyl, Halogen, gegebenenfalls substituiertes Aryl und Aralkyl oder die Gruppierungen $-OR^3$, $-SR^3$ und $NR^2R^3$ steht,

$R^2$ für Wasserstoff, Alkyl, oder gegebenenfalls substituiertes Aryl steht,

$R^3$ für Wasserstoff, Alkyl, Halogenalkyl, Alkenyl, Alkinyl, Cycloalkyl oder gegebenenfalls substituiertes Aralkyl steht,

X für Alkyl steht,

Y für Alkyl oder Halogen steht und

n für die Zahlen 0, 1 oder 2 steht,

gefunden.

Weiterhin wurde gefunden, daß man N-Azolylalkyl-aniline der Formel (I) erhält, wenn man

a) Aniline der Formel

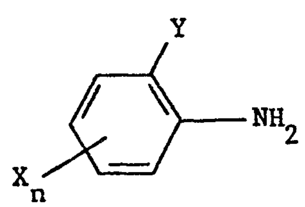

(II)

in welcher

X, Y und n die oben angegebene Bedeutung haben,

mit Azol-Derivaten der Formel

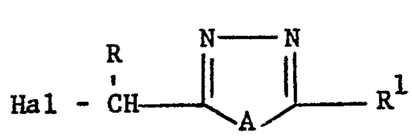

(III)

in welcher

A, R und R[1] die oben angegebene Bedeutung haben und Hal für Chlor oder Brom steht,

in Gegenwart eines Säurebinders und in Gegenwart eines Verdünnungsmittels umsetzt, oder

b) Hydrazin-Derivate der Formel

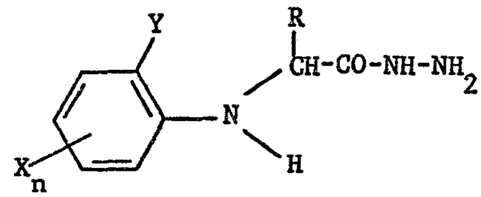

(IV)

in welcher

R, X, Y und n die oben angegebene Bedeutung haben

mit Isocyanaten bzw. Senfölen der Formel

$$R^2\text{—}N{=}C{=}B \quad (V)$$

in welcher

B für Sauerstoff oder Schwefel steht und

$R^2$ die oben angegebene Bedeutung hat,

in Gegenwart eines Verdünnungsmittels umsetzt, die dabei entstehenden Verbindungen der Formel

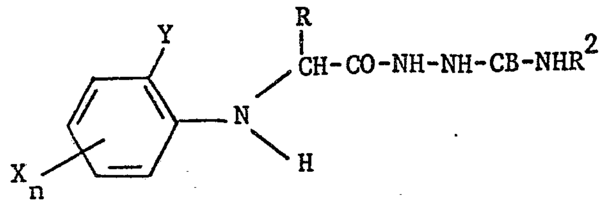

(VI)

in welcher

B, R $R^2$, X, Y und n die oben angegebene Bedeutung haben,

in Gegenwart einer starken Base und in Gegenwart eines Verdünnungsmittels cyclisiert und die dabei entstehenden Triazolone bzw. Triazolthione der Formel

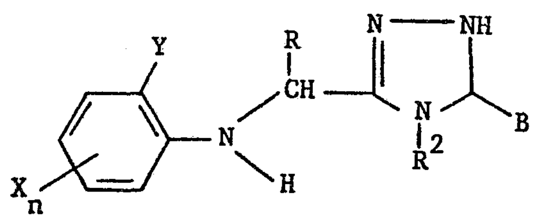

(VII)

in welcher

B, R, $R^2$, X, Y und n die oben angegebene Bedeutung haben,

mit Halogeniden der Formel

$$Hal\text{—}R^4 \quad (VIII)$$

in welcher

Hal für Chlor oder Brom steht und

$R^4$ für die Reste des Substituenten $R^3$ steht, wobei Wasserstoff ausgenommen ist,

oder mit einem anderen Alkylierungsmittel als einem solchen der Formel (VIII) jeweils in Gegenwart einer starken Base und in Gegenwart eines Verdünnungsmittels sowie gegebenenfalls in Gegenwart eines Phasen-Transferkatalysators umsetzt.

Überraschenderweise sind die N-Azolylalkyl-halogenacetanilide, die sich aus den erfindungsgemäßen N-Azolylalkylanilinen der Formel (I) durch Umsetzung mit Halogenessigsäurechloriden bzw. -anhydriden herstellen lassen, dem bekannten 2,6-Diäthyl-N-methoxymethyl-chloracetanilid bei gleichwertiger Wirkung gegen Monocotyle in ihrer Wirkung gegen Dicotyle weitgehend überlegen. Vor allem aber zeigen sie überraschenderweise eine bessere Selektivität in wichtigen Kulturpflanzen als die zuvor erwähnte, vorbekannte Verbindung, welche ein hochaktiver Wirkstoff gleicher Wirkungsart ist. Die

erfindungsgemäßen Stoffe stellen somit als Zwischenprodukte zur Synthese hochwirksamer Herbizide eine wertvolle Bereicherung der Technik dar.

Die erfindungsgemäßen N-Azolylalkyl-aniline sind durch die Formel (I) allgemein definiert. In der Formel (I) steht A vorzugsweise für Sauerstoff, Schwefel oder die Gruppierung $—NR_2$, worin $R^2$ vorzugsweise für Wasserstoff, geradkettiges oder verzweigtes Alkyl mit 1 bis 4 Kohlenstoffatomen sowie für Aryl mit 6 bis 10 Kohlenstoffatomen, insbesondere Phenyl, steht, wobei jeder dieser Arylreste substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoff- und bis zu 5 gleichen oder verschiedenen Halogenatomen, wobei als Halogene insbesondere Fluor und Chlor genannt seien. R steht vorzugsweise für Wasserstoff oder geradkettiges oder verzweigtes Alkyl mit 1 bis 4 Kohlenstoffatomen. $R^1$ steht in der Formel (I) vorzugsweise für Wasserstoff, geradkettiges oder verzweigtes Alkyl mit 1 bis 4 Kohlenstoffatomen, Halogenalkyl mit bis zu 3 Kohlenstoff- und bis zu 5 gleichen oder verschiedenen Halogenatomen, wobei als Halogene insbesondere Fluor und Chlor stehen, beispielhaft sei Trifluormethyl genannt, ferner vorzugsweise für Alkenyl und Alkinyl mit 2 bis 4 Kohlenstoffatomen, Cycloalkyl mit 5 bis 7 Kohlenstoffatomen sowie für Halogen, insbesondere Fluor, Chlor oder Brom. $R^1$ steht ferner vorzugsweise für Aryl mit 6 bis 10 Kohlenstoffatomen, insbesondere Phenyl, wobei jeder dieser Arylreste substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. $R^1$ steht ferner vorzugsweise für Aralkyl mit 6 bis 10 Kohlenstoffatomen im Arylteil und 1 bis 4 Kohlenstoffatomen im Alkylteil, insbesondere für Benzyl, wobei jeder dieser Aralkylreste im Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. Außerdem steht $R^1$ für die Gruppierung $—OR^3$, $—SR^3$ und $—NR^2R^3$, worin $R^2$ vorzugsweise für diejenigen Reste steht, die oben bereits vorzugsweise für diesen Rest genannt wurden. $R^3$ steht in diesen Gruppierungen vorzugswiese für Wasserstoff, geradkettiges oder verzweigtes Alkyl mit 1 bis 4 Kohlenstoffatomen, Halogenalkyl mit bis zu 3 Kohlenstoff- und bis zu 5 gleichen oder verschiedenen Halogenatomen, wobei als Halogene insbesondere Fluor und Chlor stehen, beispielhaft sei Trifluormethyl genannt, ferner vorzugsweise für Alkenyl und Alkinyl mit 2 bis 4 Kohlenstoffatomen, Cycloalkyl mit 5 bis 7 Kohlenstoffatomen sowie für Aralkyl mit 6 bis 10 Kohlenstoffatomen im Arylteil und 1 bis 4 Kohlenstoffatomen im Alkylteil, insbesondere für Benzyl, wobei jeder dieser Aralkylreste im Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. In der Formel (I) steht X vorzugsweise für geradkettiges oder verzweigtes Alkyl mit 1 bis 4 Kohlenstoffatomen, Y steht in der Formel (I) vorzugsweise für geradkettiges oder verzweigtes Alkyl mit 1 bis 4 Kohlenstoffatomen oder für die Halogene Fluor, Chlor und Brom, und der Index n steht für die Zahlen 0, 1 oder 2.

Als Beispiele für N-Azolylalkyl-aniline der Formel (I) seien im einzelnen genannt:

2,6-Diäthyl-N-(5-methyl-1,3,4-oxadiazol-2-yl-methyl)-anilin
2-Äthyl-6-methyl-N-(5-methyl-1,3,4-oxadiazol-2-yl-methyl)-anilin
2,6-Dimethyl-N-(5-methyl-1,3,4-oxadiazol-2-yl-methyl)-anilin
2-tert.-Butyl-N-(5-methyl-1,3,4-oxadiazol-2-yl-methyl)-anilin
2-Chlor-6-tert.-butyl-N-(5-methyl-1,3,4-oxadiazol-2-yl-methyl)-anilin
2-Chlor-3-methyl-N-(5-methyl-1,3,4-oxadiazol-2-yl-methyl)-anilin
2-Chlor-5-methyl-N-(5-methyl-1,3,4-oxadiazol-2-yl-methyl)-anilin
2-Chlor-6-isopropyl-N-(5-methyl-1,3,4-oxadiazol-2-yl-methyl)-anilin
2-Isopropyl-N-(5-methyl-1,3,4-oxadiazol-2-yl-methyl)-anilin
2-Äthyl-6-isopropyl-N-(5-methyl-1,3,4-oxadiazol-2-yl-methyl)-anilin
2-Brom-6-tert.-butyl-N-(5-methyl-1,3,4-oxadiazol-2-yl-methyl)-anilin
2-Brom-6-isopropyl-N-(5-methyl-1,3,4-ixadiazol-2-yl-methyl)-anilin
2,3-Dimethyl-N-(5-methyl-1,3,4-oxadiazol-2-yl-methyl)-anilin
2,5-Dimethyl-N-(5-methyl-1,3,4-oxadiazol-2-yl-methyl)-anilin
2,6-Diäthyl-(5-äthyl-1,3,4-oxadiazol-2-yl-methyl)-anilin
2-Äthyl-6-methyl-N-(5-äthyl-1,3,4-oxadiazol-2-yl-methyl)-anilin
2,6-Dimethyl-N-(5-äthyl-1,3,4-oxadiazol-2-yl-methyl)-anilin
2-tert.-Butyl-N-(5-äthyl-1,3,4-oxadiazol-2-yl-methyl)-anilin
2-Chlor-6-tert.-butyl-N-(5-äthyl-1,3,4-oxadiazol-2-yl-methyl)-anilin
2,6-Diäthyl-N-(5-isopropyl-1,3,4-oxadiazol-2-yl-methyl)-anilin

2-Äthyl-6-methyl-N-(5-isopropyl-1,3,4-oxadiazol-2-yl-methyl)-anilin
2,6-Dimethyl-N-(5-isopropyl-1,3,4-oxadiazol-2-yl-methyl)-anilin
2-tert.-Butyl-N-(5-isopropyl-1,3,4-oxadiazol-2-yl-methyl)-anilin
2,6-Diäthyl-N-(5-n-propyl-1,3,4-oxadiazol-2-yl-methyl)-anilin
2-Äthyl-6-methyl-N-(5-n-propyl-1,3,4-oxadiazol-2-yl-methyl)-anilin
2,6-Dimethyl-N-(5-n-propyl-1,3,4-oxadiazol-2-yl-methyl)-anilin
2-tert.-Butyl-N-(5-n-propyl-1,3,4-oxadiazol-2-yl-methyl)-anilin
2,6-Diäthyl-N-(5-phenyl-1,3,4-oxadiazol-2-yl-methyl)-anilin
2,6-Diäthyl-N-(5-phenyl-1,3,4-oxadiazol-2-yl-methyl)-anilin
2-Äthyl-6-methyl-N-(5-trichlormethyl-1,3,4-oxadiazol-2-yl-methyl)-anilin
2,6-Dimethyl-N-(5-trichlormethyl-1,3,4-oxadiazol-2-yl-methyl)-anilin
2-tert.-Butyl-N-(5-trichlormethyl-1,3,4-oxadiazol-2-yl-methyl)-anilin
2-Chlor-6-tert.-butyl-N-(5-trichlormethyl-1,3,4-oxadiazol-2-yl-methyl)-anilin
2,6-Diäthyl-N-(5-trifluormethyl-1,3,4-oxadiazol-2-yl-methyl)-anilin
2-Athyl-6-methyl-N-(5-trifluormethyl-1,3,4-oxadiazol-2-yl-methyl)-anilin
2,6-Dimethyl-N-(5-trifluormethyl-1,3,4-oxadiazol-2-yl-methyl)-anilin
2-tert.-Butyl-N-(5-trifluormethyl-1,3,4-oxadiazol-2-yl-methyl)-anilin
2-Chlor-6-tert.-butyl-N-(5-trifluormethyl-1,3,4-oxadiazol-2-yl-methyl)-anilin
2,6-Diäthyl-N-(5-chlormethyl-1,3,4-oxadiazol-2-yl-methyl)-anilin
2-Äthyl-6-methyl-N-(5-chlormethyl-1,3,4-oxadiazol-2-yl-methyl)-anilin
2,6-Dimethyl-N-(5-chlormethyl-1,3,4-oxadiazol-2-yl-methyl)-anilin
2,6-Diäthyl-N-(5-chlor-1,3,4-oxadiazol-2-yl-methyl)-anilin
2-Äthyl-6-methyl-N-(5-chlor-1,3,4-oxadiazol-2-yl-methyl)-anilin
2,6-Dimethyl-N-(5-chlor-1,3,4-oxadiazol-2-yl-methyl)-anilin
2,6-Diäthyl-N-(5-brom-1,3,4-oxadiazol-2-yl-methyl)-anilin
2-Äthyl-6-methyl-N-(5-brom-1,3,4-oxadiazol-2-yl-methyl)-anilin
2,6-Diäthyl-N-(5-methoxy-1,3,4-oxadiazol-2-yl-methyl)-anilin
2-Äthyl-6-methyl-N-(5-methoxy-1,3,4-oxadiazol-2-yl-methyl)-anilin
2,6-Dimethyl-N-(5-methoxy-1,3,4-oxadiazol-2-yl-methyl)-anilin
2-tert.-Butyl-N-(5-methoxy-1,3,4-oxadiazol-2-yl-methyl)-anilin
2,6-Diäthyl-N-(5-methylthio-1,3,4-oxadiazol-2-yl-methyl)-anilin
2-Äthyl-6-methyl-N-(5-methylthio-1,3,4-oxadiazol-2-yl-methyl)-anilin
2,6-Dimethyl-N-(5-methylthio-1,3,4-oxadiazol-2-yl-methyl)-anilin
2-tert.-Butyl-N-(5-methylthio-1,3,4-oxadiazol-2-yl-methyl)-anilin
2,6-Diäthyl-N-(5-dimethylamino-1,3,4-oxadiazol-2-yl-methyl)-anilin
2-Äthyl-6-methyl-N-(5-dimethylamino-1,3,4-oxadiazol-2-yl-methyl)-anilin
2,6-Diäthyl-N-(5-benzyl-1,3,4-oxadiazol-2-yl-methyl)-anilin
2-Äthyl-6-methyl-N-(5-benzyl-1,3,4-oxadiazol-2-yl-methyl)-anilin
2,6-Diäthyl-N-(5-methyl-1,3,4-thiadiazol-2-yl-methyl)-anilin
2-Äthyl-6-methyl-N-(5-methyl-1,3,4-thiadiazol-2-yl-methyl)-anilin
2,6-Dimethyl-N-(5-methyl-1,3,4-thiadiazol-2-yl-methyl)-anilin
2-tert.-Butyl-N-(5-methyl-1,3,4-thiadiazol-2-yl-methyl)-anilin
2-Chlor-6-tert.-Butyl-N-(5-methyl-1,3,4-thiadiazol-2-yl-methyl)-anilin
2-Chlor-3-methyl-N-(5-methyl-1,3,4-thiadiazol-2-yl-methyl)-anilin
2-Chlor-5-methyl-N-(5-methyl-1,3,4-thiadiazol-2-yl-methyl)-anilin
2-Chlor-6-isopropyl-N-(5-methyl-1,3,4-thiadiazol-2-yl-methyl)-anilin
2-Isopropyl-N-(5-methyl-1,3,4-thiadiazol-2-yl-methyl)-anilin
2-Äthyl-6-isopropyl-N-(5-methyl-1,3,4-thiadiazol-2-yl-methyl)-anilin
2-Brom-6-tert.-butyl-N-(5-methyl-1,3,4-thiadiazol-2-yl-methyl)-anilin
2-Brom-6-isopropyl-N-(5-methyl-1,3,4-thiadiazol-2-yl-methyl)-anilin
2,3-Dimethyl-N-(5-methyl-1,3,4-thiadiazol-2-yl-methyl)-anilin
2,5-Dimethyl-N-(5-methyl-1,3,4-thiadiazol-2-yl-methyl)-anilin
2,6-Diäthyl-N-(5-äthyl-1,3,4-thiadiazol-2-yl-methyl)-anilin
2-Äthyl-6-methyl-N-(5-äthyl-1,3,4-thiadiazol-2-yl-methyl)-anilin
2,6-Dimethyl-N-(5-äthyl-1,3,4-thiadiazol-2-yl-methyl)-anilin
2-tert.-Butyl-N-(5-äthyl-1,3,4-thiadiazol-2-yl-methyl)-anilin
2-Chlor-6-tert.-Butyl-N-(5-äthyl-1,3,4-thiadiazol-2-yl-methyl)-anilin
2,6-Diäthyl-N-(5-isopropyl-1,3,4-thiadiazol-2-yl-methyl)-anilin
2-Äthyl-6-methyl-N-(5-isopropyl-1,3,4-thiadiazol-2-yl-methyl)-anilin
2,6-Dimethyl-N-(5-isopropyl-1,3,4-thiadiazol-2-yl-methyl)-anilin
2-tert.-Butyl-N-(5-isopropyl-1,3,4-thiadiazol-2-yl-methyl)-anilin
2,6-Diäthyl-N-(5-n-propyl-1,3,4-thiadiazol-2-yl-methyl)-anilin
2-Äthyl-6-methyl-N-(5-n-propyl-1,3,4-thiadiazol-2-yl-methyl)-anilin
2,6-Dimethyl-N-(5-n-propyl-1,3,4-thiadiazol-2-yl-methyl)-anilin
2-tert.-Butyl-N-(5-n-propyl-1,3,4-thiadiazol-2-yl-methyl)-anilin

2,6-Diäthyl-N-(5-phenyl-1,3,4-thiadiazol-2-yl-methyl)-anilin
2,6-Diäthyl-N-(5-phenyl-1,3,4-thiadiazol-2-yl-methyl)-anilin
2-Äthyl-6-methyl-N-(5-trichlormethyl-1,3,4-thiadiazol-2-yl-methyl)-anilin
2,6-Dimethyl-N-(5-trichlormethyl-1,3,4-thiadiazol-2-yl-methyl)-anilin
2-tert.-Butyl-N-(5-trichlormethyl-1,3,4-thiadiazol-2-yl-methyl)-anilin
2-Chlor-6-tert.-butyl-N-(5-trichlormethyl-1,3,4-thiadiazol-2-yl-methyl)-anilin
2,6-Diäthyl-N-(5-trifluormethyl-1,3,4-thiadiazol-2-yl-methyl)-anilin
2-Äthyl-6-methyl-N-(5-trifluormethyl-1,3,4-thiadiazol-2-yl-methyl)-anilin
2,6-Dimethyl-N-(5-trifluoromethyl-1,3,4-thiadiazol-2-yl-methyl)-anilin
2-tert.-Butyl-N.(5-trifluormethyl-1,3,4-thiadiazol-2-yl-methyl)-anilin
2-Chlor-6-tert.-butyl-N-(5-trifluormethyl-1,3,4-thiadiazol-2-yl-methyl)-anilin
2,6-Diäthyl-N-(5-chlormethyl-1,3,4-thiadiazol-2-yl-methyl)-anilin
2-Äthyl-6-methyl-N-(5-chlormethyl-1,3,4-thiadiazol-2-yl-methyl)-anilin
2,6-Dimethyl-N-(5-chlormethyl-1,3,4-thiadiazol-2-yl-methyl)-anilin
2,6-Diäthyl-N-(5-chlor-1,3,4-thiadiazol-2-yl-methyl)-anilin
2-Äthyl-6-methyl-N-(5-chlor-1,3,4-thiadiazol-2-yl-methyl)-anilin
2,6-Dimethyl-N-(5-chlor-1,3,4-thiadiazol-2-yl-methyl)-anilin
2,6-Diäthyl-N-(5-brom-1,3,4-thiadiazol-2-yl-methyl)-anilin
2-Äthyl-6-methyl-N-(5-brom-1,3,4-thiadiazol-2-yl-methyl)-anilin
2,6-Diäthyl-N-(5-methoxy-1,3,4-thiadiazol-2-yl-methyl)-anilin
2-Äthyl-6-methyl-N-(5-methoxy-1,3,4-thiadiazol-2-yl-methyl)-anilin
2,6-Dimethyl-N-(5-methoxy-1,3,4-thiadiazol-2-yl-methyl)-anilin
2-tert.-Butyl-N-(5-methoxy-1,3,4-thiadiazol-2-yl-methyl)-anilin
2,6-Diäthyl-N-(5-methylthio-1,3,4-thiadiazol-2-yl-methyl)-anilin
2-Äthyl-6-methyl-N-(5-methylthio-1,3,4-thiadiazol-2-yl-methyl)-anilin
2,6-Dimethyl-N-(5-methylthio-1,3,4-thiadiazol-2-yl-methyl)-anilin
2-tert.-Butyl-N-(5-methylthio-1,3,4-thiadiazol-2-yl-methyl)-anilin
2,6-Diäthyl-N-(5-dimethylamino-1,3,4-thiadiazol-2-yl-methyl)-anilin
2-Äthyl-6-methyl-N-(5-dimethylamino-1,3,4-thiadiazol-2-yl-methyl)-anilin
2,6-Diäthyl-N-(5-benzyl-1,3,4-thiadiazol-2-yl-methyl)-anilin
2-Äthyl-6-methyl-N-(5-benzyl-1,3,4-thiadiazol-2-yl-methyl)-anilin
2,6-Diäthyl- N-(5-äthylthio-1,3,4-thiadiazol-2-yl-methyl)-anilin
2-Äthyl-6-methyl-N-(5-äthylthio-1,3,4-thiadiazol-2-yl-methyl)-anilin
2,6-Dimethyl-N-(5-äthylthio-1,3,4-thiadiazol-2-yl-methyl)-anilin
2-tert.-Butyl-N-(5-äthylthio-1,3,4-thiadiazol-2-yl-methyl)-anilin
2,6-Diäthyl-N-(5-allylthio-1,3,4-thiadiazol-2-yl-methyl)-anilin
2-Äthyl-6-methyl-N-(5-allylthio-1,3,4-thiadiazol-2-yl-methyl)-anilin
2,6-Dimethyl-N-(5-allylthio-1,3,4-thiadiazol-2-yl-methyl)-anilin
2-tert.-Butyl-N-(5-allylthio-1,3,4-thiadiazol-2-yl-methyl)-anilin
2,6-Diäthyl-N-(5-benzylthio-1,3,4-thiadiazo2-yl-methyl)-anilin
2-Äthyl-6-methyl-N-(5-benzylthio-1,3,4-thiadiazol-2-yl-methyl)-anilin
2,6-Dimethyl-N-(5-benzylthio-1,3,4-thiadiazol-2-yl-methyl)-anilin
2-tert.-Butyl-N-(5-benzylthio-1,3,4-thiadiazol-2-yl-methyl)-anilin
2,6-Diäthyl-N-(5-benzylthio-1,3,4-thiadiazol-2-yl-methyl)-anilin
2-Äthyl-6-methyl-N-(5-isopropylthio-1,3,4-thiadiazol-2-yl-methyl)-anilin
2,6-Dimethyl-N-(5-isopropylthio-1,3,4-thiadiazol-2-yl-methyl)-anilin
2-tert.-Butyl-N-(5-isopropylthio-1,3,4-thiadiazol-2-yl-methyl)-anilin
2,6-Diäthyl-N-(3,4-dimethyl-1,3,4-triazol-2-yl-methyl)-anilin
2-Äthyl-6-methyl-N-(3,4-dimethyl-1,2,4-triazol-5-yl-methyl)-anilin
2,6-Dimethyl-N-(3,4-dimethyl-1,2,4-triazol-5-yl-methyl)-anilin
2-tert.-Butyl-N-(3,4-dimethyl-1,2,4-triazol-5-yl-methyl)-anilin
2,6-Diäthyl-N-(3,4-dimethyl-1,3,4-triazol-5-yl-methyl)-anilin
2-Äthyl-6-methyl-N-(3-äthyl-4-methyl-1,2,4-triazol-5-yl-methyl)-anilin
2,6-Dimethyl-N-(3-äthyl-4-methyl-1,2,4-triazol-5-yl-methyl)-anilin
2-tert.-Butyl-N-(3-äthyl-4-methyl-1,2,4-triazol-5-yl-methyl)-anilin
2,6-Diäthyl-N-(3-methyl-4-phenyl-1,2,4-triazol-5-yl-methyl)-anilin
2-Äthyl-6-methyl-N-(3-methyl-4-phenyl-1,2,4-triazol-5-yl-methyl)-anilin
2,6-Dimethyl-N-(3-methyl-4-phenyl-1,2,4-triazol-5-yl-methyl)-anilin
2-tert.-Butyl-N-(3-methyl-4-phenyl-1,2,4-triazol-5-yl-methyl)-anilin
2,6-Diäthyl-N-(3-methylmercapto-4-methyl-1,2,4-triazol-5-yl-methyl)-anilin
2-Äthyl-6-methyl-N-(3-methylmercapto-4-methyl-1,2,4-triazol-5-yl-methyl)-anilin
2,6-Dimethyl-N-(3-methylmercapto-4-methyl-1,2,4-triazol-5-yl-methyl)-anilin
2-tert.-Butyl-N-(3-methylmercapto-4-methyl-1,2,4-triazol-5-yl-methyl)-anilin
2,6-Diäthyl-N-(3-methoxy-4-methyl-1,2,4-triazol-5-yl-methyl)-anilin
2-Äthyl-6-methyl-N-(3-methoxy-4-methyl-1,2,4-triazol-5-yl-methyl)-anilin

2,6-Dimethyl-N-(3-methoxy-4-methyl-1,2,4-triazol-5-yl-methyl)-anilin
2-tert.-Butyl-N-(3-methoxy-4-methyl-1,2,4-triazol-5-yl-methyl)-anilin
2,6-Diäthyl-N-(3-methylmercapto-4-phenyl-1,2,4-triazol-5-yl-methyl)-anilin
2-Äthyl-5-methyl-N-(3-methylmercapto-4-phenyl-1,2,4-triazol-5-yl-methyl)-anilin
2,6-Dimethyl-N-(3-methylmercapto-4-phenyl-1,2,4-triazol-5-yl-methyl)-anilin
2-tert.-Butyl-N-(3-methylmercapto-4-phenyl-1,2,4-triazol-5-yl-methyl)-anilin
2,6-Diäthyl-N-(3-methoxy-4-phenyl-1,2,4-triazol-5-yl-methyl)-anilin
2-Äthyl-6-methyl-N-(3-methoxy-4-phenyl-1,2,4-triazol-5-yl-methyl)-anilin
2,6-Dimethyl-N-(3-methoxy-4-phenyl-1,2,4-triazol-5-yl-methyl)-anilin
2-tert.-Butyl-N-(3-methoxy-4-phenyl-1,2,4-triazol-5-yl-methyl)-anilin
2,6-Diisopropyl-N-(5-methyl-1,3,4-oxadiazol-2-yl-methyl)-anilin
2,6-Diäthyl-N-(1,3,4-oxadiazol-2-yl-methyl)-anilin
2,6-Dimethyl-N-(1,3,4-oxadiazol-2-yl-methyl)-anilin
2-Äthyl-6-methyl-N-(1,3,4-oxadiazol-2-yl-methyl)-anilin
2,6-Diisopropyl-N-(1,3,4-oxadiazol-2-yl-methyl)-anilin
2-tert.-Butyl-N-(1,3,4-oxadiazol-2-yl-methyl)-anilin
2-Chlor-6-tert.-butyl-N-(1,3,4-oxadiazol-2-yl-methyl)-anilin
2-Brom-6-tert.-butyl-N-(1,3,4-oxadiazol-2-yl-methyl)-anilin
2,3-Dimethyl-N-(1,3,4-oxadiazol-2-yl-methyl)-anilin
2,5-Dimethyl-N-(1,3,4-oxadiazol-2-yl-methyl)-anilin
2-Chlor-6-methyl-N-(1,3,4-oxadiazol-2-yl-methyl)-anilin
2-Chlor-6-äthyl-N-(1,3,4-oxadiazol-2-yl-methyl)-anilin
2,6-Diäthyl-N-(1,3,4-thiadiazol-2-yl-methyl)-anilin
2,6-Dimethyl-N-(1,3,4-thiadiazol-2-yl-methyl)-anilin
2,6-Diisopropyl-N-(1,3,4-thiadiazol-2-yl-methyl)-anilin
2-Äthyl-6-methyl-N-(1,3,4-thiadiazol-2-yl-methyl)-anilin
2-tert.-Butyl-N-(1,3,4-thiadiazol-2-yl-methyl)-anilin
2-Chlor-6-methyl-N-(1,3,4-thiadiazol-2-yl-methyl)-anilin
2,3-Dimethyl-N-(1,3,4-thiadiazol-2-yl-methyl)-anilin
2-Chlor-6-tert.-butyl-N-(1,3,4-thiadiazol-2-yl-methyl)-anilin
2,5-Dimethyl-N-(1,3,4-thiadiazol-2-yl-methyl)-anilin
2-Brom-6-tert.-butyl-N-(1,3,4-thiadiazol-2-yl-methyl)-anilin
2,6-Diisopropyl-N-(5-methyl-1,3,4-thiadiazol-2-yl-methyl)-anilin
2,6-Diäthyl-N-(4-methyl-1,2,4-triazol-5-yl-methyl)-anilin
2,6-Dimethyl-N-(4-methyl-1,2,4-triazol-5-yl-methyl)-anilin
2,6-Diisopropyl-N-(4-methyl-1,2,4-triazol-5-yl-methyl)-anilin
2-tert.-Butyl-N-(4-methyl-1,2,4-triazol-5-yl-methyl)-anilin
2-Chlor-6-tert.-butyl-N-(4-methyl-1,2,4-triazol-5-yl-methyl)-anilin
2-Äthyl-6-methyl-(4-methyl-1,2,4-triazol-5-yl-methyl)-anilin

Verwendet man beispielsweise 2,6-Diäthylanilin und 2-Chlormethyl-5-methyl-1,3,4-oxadiazol als Ausgangsstoffe, so kann der Reaktionsablauf nach dem erfindungsgemäßen Verfahren (a) durch das folgende Formelschema wiedergegeben werden:

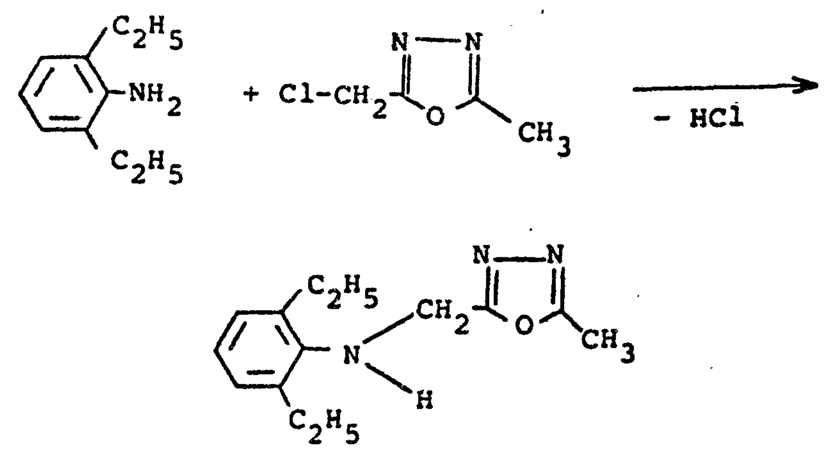


Verwendet man beispielsweise 2,6-Diäthylanilino-essigsäure-hydrazid und Phenylsenföl als Ausgangsstoffe und Dimethylsulfat als Alkylierungsmittel sowie Triäthyl-benzyl-ammoniumchlorid als Phasentransfer-Katalysator, so kann der Verlauf des erfindungsgemäßen Verfahrens (b) durch das folgende Formelschema wiedergegeben werden

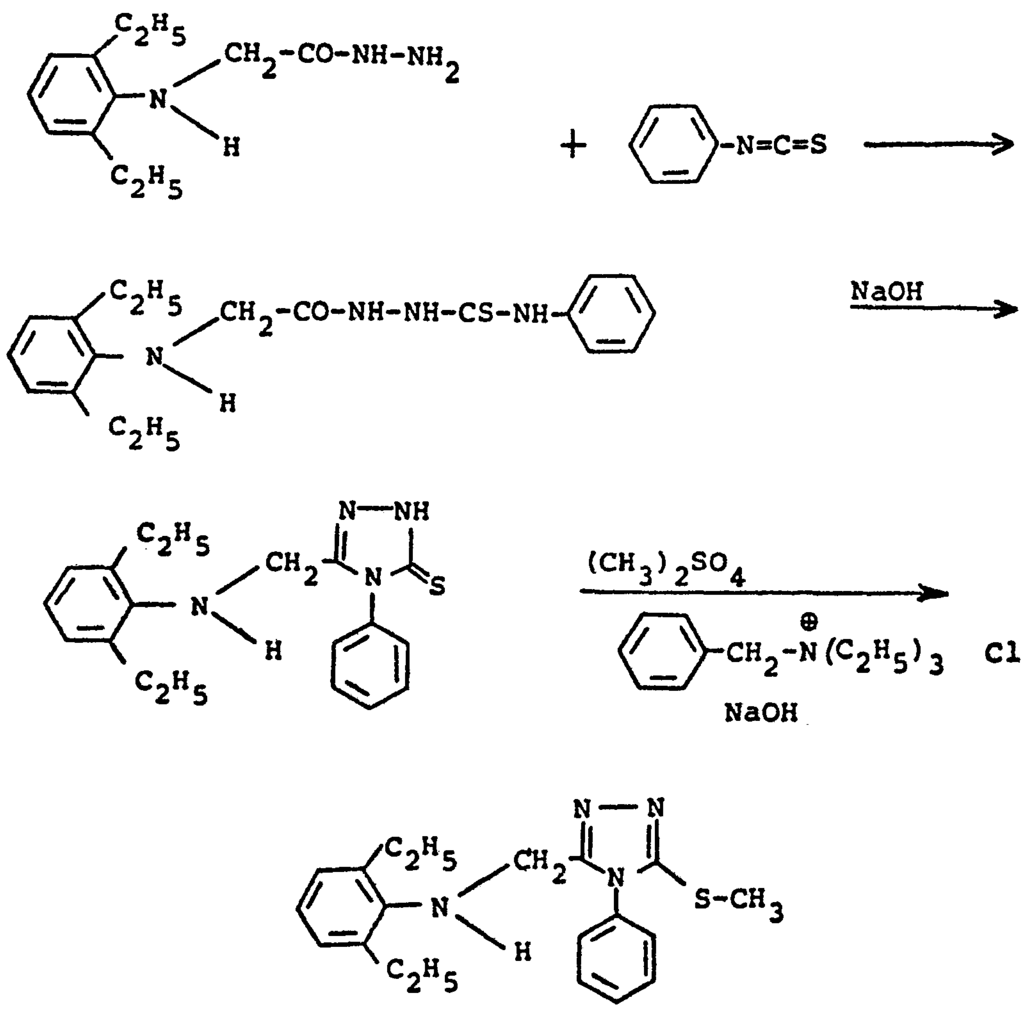


Die bei der Durchführung des erfindungsgemäßen Verfahrens (a) als Ausgangsstoffe benötigten Aniline sind durch die Formel (II) allgemein definiert. In der Formel (II) stehen X, Y und n vorzugsweise für diejenigen reste, die bereits im Zusammenhang mit der Beschreibung der erfindungsgemäßen Stoffe der Formel (I) vorzugsweise für X, Y und n genannt wurden.

Die Verbindungen der Formel (II) sind bekannt. Als Beispiele seien im einzelnen genannt: 2-Methylanilin, 2-Äthylanilin, 2-Propylanilin, 2-Isopropylanilin, 2-Butylanilin, 2-Isobutylanilin, 2-sec.-Butylanilin, 2-tert.-Butylanilin, 2,6-Dimethylanilin, 2,6-Diäthylanilin, 2-Äthyl-6-methylanilin, 2,6-Diisopropylanilin, 2,3-Dimethylanilin, 2,4-Dimethylanilin, 2,5-Dimethylanilin, 2,6-Dimethylanilin, 2-Äthyl-3-methylanilin, 2-Äthyl-4-methylanilin, 2-Äthyl-5-methylanilin, 2,4,6-Trimethylanilin, 2,4,5-Trimethylanilin, 2-Äthyl-4,6-dimethylanilin, 2,6-Diäthyl-4-methylanilin, 2,6-Diisopropyl-4-methylanilin, 2-Chlor-6-methylanilin, 5-Chlor-2-methyl-anilin, 3-Chlor-2-methylanilin, 4-Chlor-2-methylanilin, 2-Chlor-6-tert.-butylanilin.

Die bei der Durchführung des erfindungsgemäßen Verfahrens (a) weiterhin als Ausgangsstoffe benötigten Azol-Derivate sind durch die Formel (III) allgemein definiert. In dieser Formel (III) steht A vorzugsweise für Sauerstoff, Schwefel oder die Gruppierung $NR^2$, worin $R^2$ vorzugsweise für Wasserstoff, geradkettiges oder verzweigtes Alkyl mit 1 bis 4 Kohlenstoffatomen sowie für Aryl mit 6 bis 10 Kohlenstoffatomen, insbesondere Phenyl, steht, wobei jeder dieser Arylreste substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoff- und bis zu 5 gleichen oder verschiedenen Halogenatomen, wobei als Halogene insbesondere Fluor und Chlor genannt seien. $R^1$ steht in der Formel (III) vorzugsweise für Wasserstoff, geradkettiges oder verzweigtes Alkyl mit 1 bis 4 Kohlenstoffatomen, Halogenalkyl mit bis zu 3 Kohlenstoff- und bis zu 5 gleichen oder verschiedenen Halogenatomen, wobei als Halogene insbesondere Fluor und Chlor stehen, beispielhaft sei Trifluormethyl genannt, ferner vorzugsweise für Alkenyl und Alkinyl mit 2 bis 4 Kohlenstoffatomen, Cycloalkyl mit 5 bis 7 Kohlenstoffatomen sowie für Halogen, insbesondere Fluor, Chlor oder Brom. $R^1$ steht ferner vorzugsweise für Aryl mit 6 bis 10 Kohlenstoffatomen, insbesondere Phenyl, wobei jeder dieser Arylreste substituiert sein kann durch Halogen, Alkyl mit 1 bis 4 Kohlenstoffatomen, Alkoxy mit 1

oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. $R^1$ steht ferner vorzugsweise für Aralkyl mit 6 bis 10 Kohlenstoffatomen im Arylteil und 1 bis 4 Kohlenstoffatomen im Alkylteil, insbesondere für Benzyl, wobei jeder dieser Aralkylreste im Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. Außerdem steht $R^1$ für die Gruppierungen $-OR^3$ $-SR^3$ und $-NR^2R^3$, worin $R^2$ vorzugsweise für diejenigen Reste steht, die oben bereits vorzugsweise für diesen Rest genannt wurden. $R^3$ steht in diesen Gruppierungen vorzugsweise für Wasserstoff, geradkettiges oder verzweigtes Alkyl mit 1 bis 4 Kohlenstoffatomen, Halogenalkyl mit bis zu 3 Kohlenstoff- und bis zu 5 gleichen oder verschiedenen Halogenatomen, wobei als Halogene insbesondere Fluor und Chlor stehen, beispielhalft sei Trifluormethyl genannt, ferner vorzugsweise für Alkenyl und Alkinyl mit 2 bis 4 Kohlenstoffatomen, Cycloalkyl mit 5 bis 7 Kohlenstoffatomen sowie für Aralkyl mit 6 bis 10 Kohlenstoffatomen in Arylteil und 1 bis 4 Kohlenstoffatomen im Alkylteil, insbesondere für Benzyl, wobei jeder dieser Aralkylreste im Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano- Nitro und/or.er Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. R steht vorzugsweise für geradkettiges oder verzweigtes Alkyl mit 1 bis 4 Kohlenstoffatomen oder für Wasserstoff; Hal steht für Chlor oder Brom.

Die Azol-Derivate der Formel (III) sind bekannt oder lassen sich nach allgemein üblichen und bekannten Heterocyclensynthesen herstellen (vergleiche hierzu u.a. Helv. Chim. Acta *55*, 1979 ff (1972); Chem. Ber. *32*, 797 ff (1899) sowie *96*, 1049 ff (1963)). Als Beispiele seien genannt:

2-Chlormethyl-5-methyl-1,3,4-oxadiazol
2-Chlormethyl-5-äthyl-1,3,4-oxadiazol
2-Chlormethyl-5-isopropyl-1,3,4-oxadiazol
2-Chlormethyl-5-trifluormethyl-1,3,4-oxadiazol
2-Chlormethyl-5-trichlormethyl-1,3,4-oxadiazol
2-Chlormethyl-5-allyl-1,3,4-oxadiazol
2-Chlormethyl-5-cyclohexyl-1,3,4-oxadiazol
2-Chlormethyl-5-benzyl-1,3,4-oxadiazol
2-Chlormethyl-5-methoxy-1,3,4-oxadiazol
2-Chlormethyl-5-allyloxy-1,3,4-oxadiazol
2-Chlormethyl-5-(2-fluorbenzyloxy)-1,3,4-oxadiazol
2-Chlormethyl-5-methylthio-1,3,4-oxadiazol
2-Chlormethyl-5-allylthio-1,3,4-oxadiazol
2-Chlormethyl-5-(2-fluorbenzylthio)-1,3,4-oxadiazol
2-(1-Chloräthyl)-5-methyl-1,3,4-oxadiazol
2-(1-Chloräthyl)-5-äthyl-1,3,4-oxadiazol
2-Chlormethyl-5-methyl-1,3,4-thiadiazol
2-Chlormethyl-5-äthyl-1,3,4-thiadiazol
2-Chlormethyl-5-isopropyl-1,3,4-thiadiazol
2-Chlormethyl-5-trifluormethyl-1,3,4-thiadiazol
2-Chlormethyl-5-trichlormethyl-1,3,4-thiadiazol
2-Chlormethyl-5-allyl-1,3,4-thiadiazol
2-Chlormethyl-5-cyclohexyl-1,3,4-thiadiazol
2-Chlormethyl-5-benzyl-1,3,4-thiadiazol
2-Chlormethyl-5-methoxy-1,3,4-thiadiazol
2-Chlormethyl-5-allyloxy-1,3,4-thiadiazol
2-Chlormethyl-5-(2-fluorbenzyloxy)-1,3,4-thiadiazol
2-Chlormethyl-5-methylthio-1,3,4-thiadiazol
2-Chlormethyl-5-allylthio-1,3,4-thiadiazol
2-Chlormethyl-5-(2-fluorbenzylthio)-1,3,4-thiadiazol
2-(1-Chloräthyl)-5-methyl-1,3,4-thiadiazol
2-(1-Chloräthyl)-5-äthyl-1,3,4-thiadiazol
2-Chlormethyl-5-methyl-1,3,4-triazol
2-Chlormethyl-5-äthyl-1,3,4-triazol
2-Chlormethyl-5-isopropyl-1,3,4-triazol
2-Chlormethyl-5-trifluormethyl-1,3,4-triazol
2-Chlormethyl-5-trichlormethyl-1,3,4-triazol
2-Chlormethyl-5-allyl-1,3,4-triazol

2-Chlormethyl-5-cyclohexyl-1,3,4-triazol
2-Chlormethyl-5-benzyl-1,3,4-triazol
2-Chlormethyl-5-methoxy-1,3,4-triazol
2-Chlormethyl-5-allyloxy-1,3,4-triazol
2-Chlormethyl-5-(2-fluorbenzyloxy)-1,3,4-triazol
2-Chlormethyl-5-methylthio-1,3,4-triazol
2-Chlormethyl-5-allylthio-1,3,4-triazol
2-Chlormethyl-5-(2-fluorbenzylthio)-1,3,4-triazol
2-(1-Chloräthyl)-5-methyl-1,3,4-triazol
2-(1-Chloräthyl)-5-äthyl-1,3,4-triazol
2-Chlormethyl-1,5-dimethyl-1,3,4-triazol
2-Chlormethyl-5-äthyl-1-methyl-1,3,4-triazol
2-Chlormethyl-5-isopropyl-1-methyl-1,3,4-triazol
2-Chlormethyl-1-methyl-5-trifluormethyl-1,3,4-triazol
2-Chlormethyl-1-methyl-5-trichlormethyl-1,3,4-triazol
2-Chlormethyl-5-allyl-1-methyl-1,3,4-triazol
2-Chlormethyl-5-cyclohexyl-1-methyl-1,3,4-triazol
2-Chlormethyl-5-benzyl-1-methyl-1,3,4-triazol
2-Chlormethyl-5-methoxy-1-methyl-1,3,4-triazol
2-Chlormethyl-5-allyloxy-1-methyl-1,3,4-triazol
2-Chlormethyl-5-(2-fluorbenzyloxy)-1-methoxy-1,3,4-triazol
2-Chlormethyl-5-methylthio-1-methyl-1,3,4-triazol
2-Chlormethyl-5-allylthio-1-methyl-1,3,4-triazol
2-Chlormethyl-5-(2-fluorbenzylthio)-1-methyl-1,3,4-triazol
2-(1-Chloräthyl)-1,5-dimethyl-1,3,4-triazol
2-(1-Chloräthyl)-5-äthyl-1-methyl-1,3,4-triazol

Als Säurebindemittel können bei der Durchführung des erfindungsgemäßen Verfahrens (a) alle üblicherweise verwendbaren Säureakzeptoren eingesetzt werden. Hierzu gehören vorzugsweise Alkalicarbonate, wie Natrium- oder Kaliumcarbonat.

Als Verdünnungsmittel kommen bei der Umsetzung nach dem erfindungsgemäßen Verfahren (a) alle üblichen inerten organischen Lösungsmittel in Betracht. Hierzu gehören vorzugsweise Kohlenwasserstoffe, wie Benzol, Toluol und Xylol, sowie polare Lösungsmittel, wie Dimethylformamid.

Die Reaktionstemperaturen können bei dem erfindungsgemäßen Verfahren (a) in einem größeren Bereich variiert werden. Im allgemeinen arbeitet man bei Temperaturen zwischen 20°C und 160°C, vorzugsweise zwischen 60°C und 120°C.

Bei der Durchführung des erfindungsgemäßen Verfahrens (a) setzt man auf 1 Mol an Azolderivat der Formel (III) vorzugsweise 1 Mol oder auch einen größeren Überschuß an Anilinen der Formel (II) sowie 1 Mol oder einen Überschuß an Säurebindemittel ein. Die Isolierung der Reaktionsprodukte erfolgt nach üblichen Methoden.

Die bei der Durchführung des erfindungsgemäßen Verfahrens (b) als Ausgangsstoffe benötigten Hydrazin-Derivate sind durch die Formel (IV) allgemein definiert. In dieser Formel steht R vorzugsweise für Wasserstoff und geradkettiges oder verzweigtes Alkyl mit 1 bis 4 Kohlenstoffatomen. X steht vorzugsweise für geradkettiges oder verzweigtes Alkyl mit 1 bis 4 Kohlenstoffatomen; Y steht vorzugsweise für geradkettiges oder verzweigtes Alkyl mit 1 bis 4 Kohlenstoffatomen oder für die Halogene Fluor, Chlor und Brom; der Index n steht für die Zahlen 0, 1 oder 2.

Als Beispiele für Hydrazin-Derivate der Formel (IV) seien im einzelnen genannt:

2,6-Diäthylanilino-essigsäure-hydrazid
2,6-Dimethylanilino-essigsäure-hydrazid
2,4,6-Trimethylanilino-essigsäure-hydrazid
2-Äthyl-6-methylanilino-essigsäure-hydrazid
α-(2,6-Diäthylanilino)-propionsäure-hydrazid
α-(2,6-Dimethylanilino-propionsäure-hydrazid
α-(2,4,6-Trimethylanilino)-propionsäure-hydrazid
α-(2-Äthyl-6-methylanilino-propionsäure-hydrazid

Die Hydrazin-Derivate der Formel (IV) sind noch nicht bekannt.

Sie lassen sich jedoch nach gekannten Verfahren herstellen, indem man bekannte Ester (vgl. u.a. DT-OS 2 350 944 und 2 513 730) der Formel

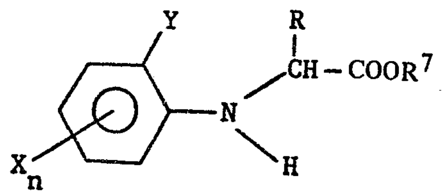

(IX)

in welcher

R, X, Y und n die oben angegebene Bedeutung haben und

$R^7$ für Methyl oder Äthyl steht,

mit Hydrazinhydrat in Gegenwart eines inerten organischen Lösungsmittel, wie beispielsweise Acetonitril oder Dimethylformamid, bei Temperaturen zwischen 20 und 80°C umsetzt (vergleiche auch Herstellungsbeispiele).

Die bei der Durchführung des erfindungsgemäßen Verfahrens (b) weiterhin als Ausgangsstoffe benötigten Isocyanate bzw. Senföle sind durch die Formel (V) allgemein definiert. In dieser Formel steht $R^2$ vorzugsweise für diejenigen Reste, die bereits im Zusammenhang mit der Beschreibung der Verbindungen der Formel (III) vorzugsweise für $R^2$ genannt wurden. B steht in der Formel (V) für Sauerstoff oder Schwefel.

Die Verbindungen der Formel (V) sind bekannt. Als Beispiele seien im einzelnen genannt:

Methylsenföl
Phenylisocyanat
Phenylsenföl
Methylisocyanat
Äthylisocyanat

Die bei der Durchführung des erfindungsgemäßen Verfahrens (b) als Reaktionskomponenten benötigten Halogenide sind durch die Formel (VIII) allgemein definiert. In dieser Formel steht $R^4$ vorzugsweise für diejenigen Reste, die bereits im Zusammenhang mit der Beschreibung der Verbindungen der Formel (III) vorzugsweise für $R^3$ genannt wurden, wobei jedoch Wasserstoff ausgenommen ist. Hal steht für Chlor oder Brom.

Die Verbindungen der Formel (VIII) sind bekannt. Als Beispiele seien im einzelnen genannt:

Äthylbromid
Propylbromid
Phenylbromid
Cyclohexylbromid
Allylbromid

Anstelle eines Halogenides der Formel (VIII) kann bei dem erfindungsgemäßen Verfahren (b) auch mit anderen Alkylierungsmitteln, wie zum Beispiel Dimethylsulfat, und einem Phasentransfer-Katalysator, wie zum Beispiel Triäthylbenzyl-ammoniumchlorid, gearbeitet werden.

Bei der Durchführung des erfindungsgemäßen Verfahrens (b) kommt bei der Umsetzung von Hydrazin-Derivaten der Formel (IV) mit Isocyanaten bzw. Senfölen der Formel (V) als Verdünnungsmittel eine breite Palette von organischen Losungsmitteln in Frage. Hierzu gehören vorzugsweise Alkohole, wie Methanol oder Äthanol und Äther, wie Dioxan oder Tetrahydrofuran. Bei der nachfolgenden Cyclisierung der zunächst entstehenden Verbindungen der Formel (VI) kommen als Verdünnungsmittel alle polaren Lösungsmittel in Betracht. Hierzu gehören vorzugsweise Alkohole, wie Methanol oder Äthanol, und ferner Wasser. Bei der anschließenden Umsetzung der durch Cyclisierung entstandenen Triazolone bzw. Triazolthione der Formel (VII) mit Halogeniden der Formel (VIII) bzw. mit anderen Alkylierungsmitteln kommen als Verdünnungsmittel alle inerten organischen Lösungsmittel in Frage. Hierzu gehören vorzugsweise Kohlenwasserstoffe, wie Benzol, Toluol und Xylol, ferner chlorierte Kohlenwasserstoffe, wie Methylenchlorid und Chloroform.

Als Basen kommen sowohl bei der Cyclisierung der Verbindungen der Formel (VI) als auch bei der Umsetzung der Triazolone bzw. Triazolthione der Formel (VII) mit Halogeniden der Formel (VIII) oder mit anderen Alkylierungsmitteln alle starken organischen oder anorganischen Basen in Betracht. Hierzu gehören vorzugsweise Alkalihydroxide, wie Natriumhydroxid oder Kaliumhydroxid.

Dir Reaktionstemperaturen können bei der Durchführung des erfindungsgemäßen Verfahrens (b) innerhalb eines größeren Bereiches variiert werden. Bei der Umsetzung von Hydrazin-Derivaten der Formel (IV) mit Isocyanaten bzw. Senfölen der Formel (V) arbeitet man im allgemeinen bei Temperaturen zwischen 0°C und 80°C, vorzugsweise zwischen 20°C und 50°C. Bei der Cyclisierung der Verbindungen der Formel (VI) arbeitet man im allgemeinen bei Temperaturen zwischen 20°C und 100°C, vorzugsweise zwischen 20°C und 80°C. Bei der Umsetzung der Triazolone bzw. Triazolthione der Formel (VII) mit Halogeniden der Formel (VIII) oder mit anderen Alkylierungsmitteln arbeitet man im

allgemeinen bei Temperaturen zwischen 20°C und 80°C, vorzugsweise zwischen 20°C und 60°C.

Bei der Durchführung des erfindungsgemäßen Verfahrens (b) setzt man auf 1 Mol Hydrazin-Derivat der Formel (IV) vorzugsweise 1 Mol an Isocyanat bzw. Senföl der Formel (V) ein, behandelt dann das dabei entstehende Produkt der Formel (VI) nach vorheriger Isolierung mit einer äquimolaren Menge oder mit einem geringen Überschuß an einer starken Base, isoliert das dabei entstehende Triazolon bzw.

Triazolthion der Formel (VII) und setzt es mit einer äquimolaren Menge oder mit einem geringen Überschuß an Halogenid der Formel (VIII) oder einem anderen Alkylierungsmittel um. Die Isolierung der Reaktionsprodukte erfolgt jeweils nach üblichen Methoden.

Die erfindungsgemäßen N-Azolylalkyl-aniline der Formel (I) eignen sich als Zwischenprodukte zur Synthese von N-Azolylalkyl-halogenacetaniliden, welche herbizide Eigenschaften besitzen. Die N-Azolylalkyl-halogenacetanilide der Formel

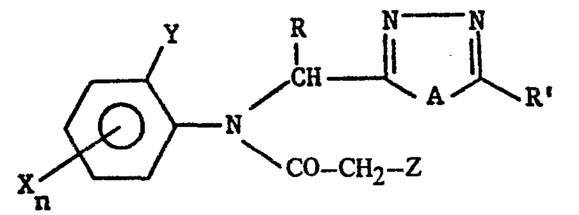

(X)

in welcher

R, $R^1$, A, Y, X und n die oben angegebene Bedeutung haben und

Z für Halogen steht,

lassen sich herstellen, indem man N-Azolylalkylaniline der Formel

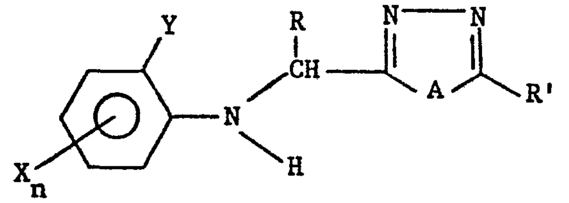

(I)

in welcher

R, $R^1$, A, X, Y und n die oben angegebene Bedeutung haben,

mit Halogenessigsäurechloriden bzw. -anhydriden der Formeln

$$Z—CH_2—CO—Cl \quad (XIa)$$

bzw.

$$(Z—CH_2—CO)_2O \quad (XIb)$$

in welchen

Z die oben angegebene Bedeutung hat,

in Gegenwart eines Verdünnungsmittels und gegebenenfalls in Gegenwart eines Säurebindemittels umsetzt.

Die bei dieser Umsetzung als Ausgangsstoffe zu verwendenden Halogenessigsäurechloride bzw. -anhydride sind durch die Formeln (XIa) und (XIb) allgemein definiert. In diesen Formeln steht Z vorzugsweise für Chlor, Brom und Jod.

Die Halogenessigsäurechloride und -anhydride der Formeln (XIa) und (XIb) sind allgemein bekannte Verbindungen der organischen Chemie. Als Beispiele seien genannt: Chloracetylchlorid, Bromacetylchlorid, Jodacetylchlorid und die entsprechenden Anhydride.

Als Verdünnungsmittel kommen bei der Umsetzung der erfindungsgemäßen N-Azolalkyl-aniline mit Halogenessigsäurechloriden bzw. -anhydriden der Formeln (XIa) und (XIb) alle inerten organischen Lösungsmittel in Frage. Hierzu gehören vorzugsweise Ketone, wie Diäthylketon, insbesondere Aceton und Methyläthylketon; Nitrile, wie Propionitril, insbesondere Acetonitril; Äther, wie Tetrahydrofuran oder Dioxan; aliphatische und aromatische Kohlenwasserstoffe, wie Petroläther, Benzol, Toluol oder Xylol; halogenierte Kohlenwasserstoffe, wie Methylenchlorid, Tetrachlorkohlenstoff, Chloroform oder Chlorbenzol, und außerdem Ester, wie Essigester.

Das oben angegebene Verfahren zur Herstellung von N-Azolylalkyl-halogenacetaniliden kann gegebenenfalls in Gegenwart von Säurebindern (Chlorwasserstoff-Akzeptoren) durchgeführt werden.

Als solche können alle üblichen Säurebindemittel verwendet werden. Hierzu gehören vorzugsweise organische Basen, wie tertiäre Amine, beispielsweise Triäthylamin, oder wie Pyridin; ferner anorganische Basen, wie beispielsweise Alkalihydroxide und Alkalicarbonate.

Die Reaktionstemperaturen können bei der Durchführung des oben angegebenen Verfahrens zur Herstellung von N-Azolylalkyl-halogenacetaniliden in einem größeren Bereich variiert werden. Im Allgemeinen arbeitet man zwischen 0 und 120°C, vorzugsweise zwischen 20 und 100°C.

Bei der Durchführung des oben angegebenen Verfahrens setzt man vorzugsweise auf 1 Mol der Verbindung der Formel (I) 1 bis 1,5 Mol Halogenacetylierungsmittel und 1 bis 1,5 Mol Säurebinder ein. Die Isolierung der Verbindungen der Formel (X) erfolgt in üblicher Weise.

Die N-Azolylalkyl-halogenacetanilide der Formel (X) zeichnen sich neben einer guten Totalherbizid-Wirkung vor allem durch ihre selektiven Einsatzmöglichkeiten in wichtigen Kulturpflanzen, wie Baumwolle, Rüben, Raps und Getreide aus. Sie eignen sich vornehmlich zur preemergence-Anwendung, aber auch bei post-emergence-Anwendung zeigen sie Wirkung.

Die guten herbiziden Wirkungen der N-Azolylalkyl-halogenacetanilide der Formel (X) gehen aus dem nachfolgenden Beispiel hervor.

*Wirkstoffliste*

In dem nachfolgenden Beispiel werden die nachstehend angegebenen Wirkstoffe bezüglich ihrer herbiziden Wirkung getestet.

(A) = 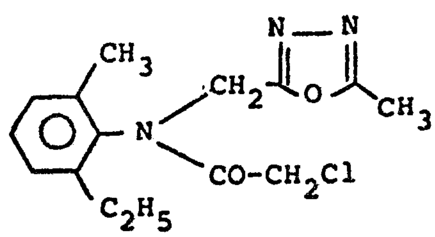

(B) = 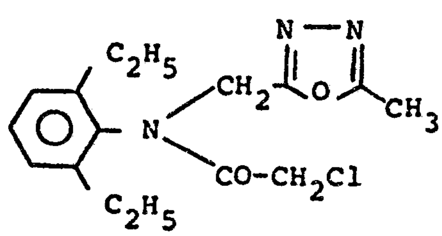

(C) = 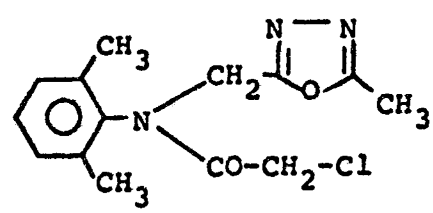

(D) = 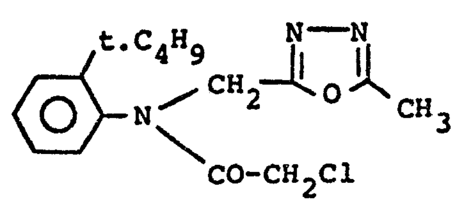

(E) = 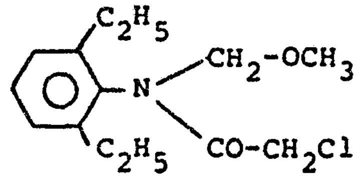

Beispiel A

Pre-emergence-Test

Lösungsmittel: 5 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Die N-Azolylalkyl-halogenacetanilide der Formeln (A) bis (D) zeigen in diesem Test eine bessere selektive herbizide Wirksamkeit als die aus dem Stand der Technik bekannte Substanz (E).

*Herstellungsbeispiele*

Beispiel 1

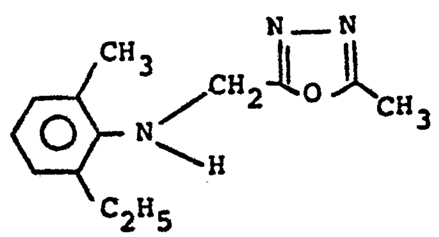

Eine Mischung aus 101,2 g (0,76 Mol) 2-Äthyl-6-methyl-anilin, 40 g (0,3 Mol) 5-Chlormethyl-1,3,4-oxadiazol, 41,4 g (0,3 Mol) gepulvertes Kaliumcarbonat und 76 ml Dimethylformamid wird 5 Stunden unter Rühren auf 100°C erhitzt. Danach wird die Reaktionsmischung filtriert, das Filtrat mit Methylenchlorid verdünnt und mehrmals mit Wasser gewaschen. Die Methylenchloridphase wird über Natriumsulfat getrocknet und im Vakuum durch Abdestillieren des Lösungsmittels eingeengt. Der Rückstand wird im Vakuum destilliert. Man erhält 46,8 g (67,5% der Theorie) gelbliches Öl von 2-Äthyl-6-methyl-N-[(2-methyl-1,3,4-oxadiazol-5-yl)-methyl]-anilin vom Siedepunkt 140—142°C/0,1 mm mit einer 94%igen Reinheit (Gaschromatografisch bestimmt).

Umsetzung der Verbindung (1) zu dem entsprechenden N-Azolylalkyl-halogenacetanilid der Formel

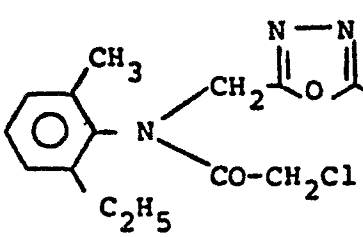

16,3 g (0,07 Mol) 2-Äthyl-6-methyl-N[(2-methyl-1,3,4-oxadiazol-5-yl)-methyl]-anilin und 6 g (0,076 Mol) wasserfreies Pyridin werden in 100 ml absolutem Tetrahydrofuran unter Rühren bis zum Sieden erhitzt und tropfenweise mit einer Lösung von 8 g (0,07 Mol) Chloracetylchlorid in 20 ml Tetrahydrofuran versetzt. Nach beendetem Zutropfen läßt man 10 Minuten nachrühren, engt durch Abdestillieren des Lösungsmittels ein und verrührt den Rückstand mit 150 ml Wasser. Das auskristallisierende Reaktionsprodukt wird abgesaugt, mit Wasser gewaschen und getrocknet. Man erhält 18,7 g (87% der Theorie) beigefarbene Kristalle von 2-Äthyl-6-methyl-N-[(2-methyl-1,3,4-oxadiazol-5-yl)-methyl]-chloracetanilid vom Schmelzpunkt 67—70°C.

Beispiel 2

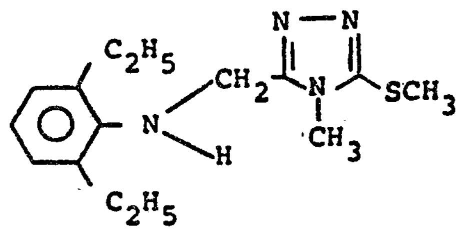

13,9 g (0,05 Mol) 2,6-Diäthyl-N-[(1-methyl-2-thiono-1,3,4-triazol-5-yl)-methyl]-anilin werden bei Raumtemperatur in einem Zweiphasengemisch aus 150 ml Toluol und 40 ml 50%iger Natronlauge unter Zusatz von 1,5 g Triäthyl-benzyl-ammoniumchlorid (TEBA) als Katalysator schnell gerührt und tropfenweise mit 6,3 g (0,05 Mol) Dimethylsulfat versetzt, wobei die Temperatur auf ca. 35°C ansteigt. Man läßt 5 Stunden rühren, trennt die Toluolphase ab, wäscht sie mehrmals mit Wasser, trocknet über Natriumsulfat und engt durch Abdestillieren des Lösungsmittels ein. Des zurückbleibende Öl wird durch Zusatz von Petroläther zur Kristallisation gebracht. Man erhält nach Umkristallisation aus Petroläther 6,7 g (40% der Theorie) 2,6-Diäthyl-N-[(1-methyl-2-methylthio-1,3,4-triazol-5-yl)-methyl]-anilin vom Schmelzpunkt 65—67°C.

Herstellung der Vorstufen:

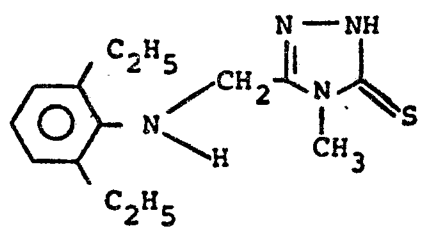

29,6 g (0,1 Mol) 1-Methyl-4-[(2,6-diäthyl-anilino)-acetyl]-thiosemicarbazid werden in 150 ml Äthanol suspendiert und nach Zugabe von 7 g Kaliumhydroxid in 20 ml Wasser 1 Stunde unter Rückfluß erhitzt. Danach wird der größte Teil des Lösungsmittels abdestilliert und der Rückstand mit 250 ml Wasser versetzt. Nach den Ansäuern mit Eisessig auf pH 5 wird der entstehende Niederschlag abgesaugt und gründlich mit Wasser gewaschen. Nach dem Trocknen erhält man 27 g (97% der Theorie) 2,6-Diäthyl-N-[(1-methyl-2-thiono-1,3,4-triazol-5-yl)-methyl]-anilin vom Schmelzpunkt 117—121°C.

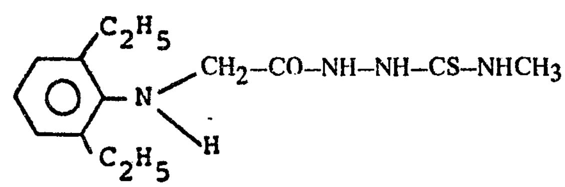

44,2 g (0,2 Mol) 2,6-Diäthyl-anilino-essigsäure-hydrazid und 14,8 g (0,2 Mol) Methylsenföl werden in 250 ml Äthanol gelöst und eine Stunde auf Rückflußtemperatur erhitzt. Nach dem anschließenden Abkühlen auf Raumtemperatur wird der entstandene Niederschlag abgesaugt und zweimal mit je 50 ml Äthanol nachgewaschen. Nach dem Trocknen erhält man 46 g (78% der Theorie) an 1-Methyl-4-[2,6-diäthyl-anilino)-acetyl]-thiosemicarbazid in Form einer farblosen kristallinen Substanz vom Schmelzpunkt 166°C.

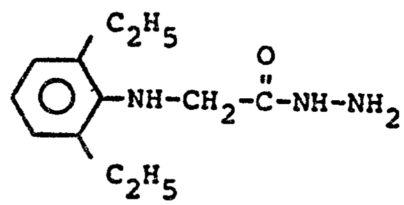

58,7 g (0,25 Mol) 2,6-Diäthyl-anilino-essigsäureäthylester und 25 g Hydrazinhydrat werden in 200 ml Äthanol 24 Stunden stehen gelassen. Danach wird durch Abdestillieren des Lösungsmittels eingeengt und der Rückstand mit Wasser ausgerührt. Nach dem Trocknen erhält man 50,5 g (91% der Theorie) farblose Kristalle von 2,6-Diäthylanilino-essigsäurehydrazid vom Schmelzpunkt 71—73°C.

Umsetzung der Verbindung (2) zu dem entsprechenden N-Azolylalkyl-halogenacetanilid der Formel

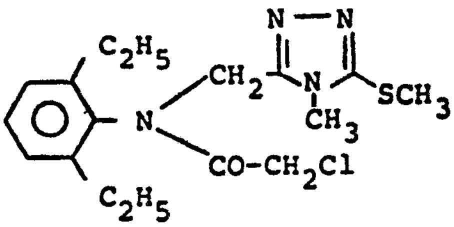

5 g (0,017 Mol) 2,6-Diäthyl-N-[(1-methyl-2-methylthio-1,3,4-triazol-5-yl)-methyl]-anilin und 1,6 g (0,02 Mol) Pyridin werden in 100 ml absolutem Tetrahydrofuran gerührt und bei Raumtemperatur tropfenweise mit 2,3 g (0,02 Mol) Chloracetylchlorid versetzt, wobei die Temperatur auf ca. 30°C ansteigt. Man läßt 2 Stunden rühren, engt teilweise durch Abdestillieren des Lösungsmittels ein und versetzt mit Wasser. Das auskristallisierende Produkt wird abgesaugt, getrocknet und aus Diisopropyläther/Essigester umkristallisiert. Man erhält 5 g (80% der Theorie) 2,6-Diäthyl-N-[(1-methyl-2-methylthio-1,3,4-triazol-5-yl)-methyl]-chloracetanilid vom Schmelzpunkt 121—123°C.

In entsprechender Weise werden diejenigen Verbindungen erhalten, die in der Tabelle 1 formelmäßig aufgeführt sind.

TABELLE 1

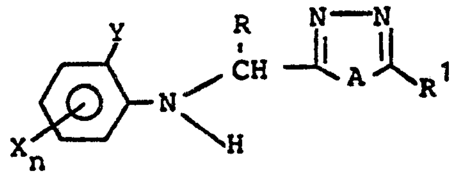

| Beisp.-Nr. | R | R¹ | Y | $X_n$ | A | Schmelzpunkt (°C) bzw. Brechungsindex |
|---|---|---|---|---|---|---|
| 3 | H | $CH_3$ | $C_2H_5$ | $6-C_2H_5$ | O | $n_D^{22} = 1,540$ |
| 4 | H | $CH_3$ | $CH_3$ | $6-CH_3$ | O | $n_D^{22} = 1,552$ |
| 5 | H | $CH_3$ | t.-$C_4H_9$ | – | O | 52–55 |
| 6 | H | $-S-CH_2-CH=CH_2$ | $C_2H_5$ | $6-C_2H_5$ | $>N-CH_3$ | $n_D^{21} = 1,577$ |
| 7 | H | 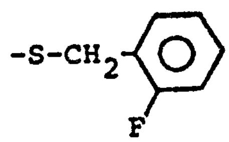 | $CH_3$ | $6-C_2H_5$ | $>N-CH_3$ | rohes Öl |
| 8 | H | $C_2H_5$ | $CH_3$ | $6-C_2H_5$ | O | $n_D^{21} = 1,542$ |
| 9 | H | $C_2H_5$ | $C_2H_5$ | $6-C_2H_5$ | O | $n_D^{22} = 1,534$ |
| 10 | H | i.-$C_3H_7$ | $CH_3$ | $6-C_2H_5$ | O | $n_D^{21} = 1,531$ |
| 11 | H | $CH_3$ | $CH_3$ | $3-CH_3$ | 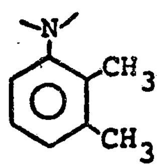 | 142–143 |
| 12 | H | $CH_3$ | i-$C_3H_7$ | 6-i-$C_3H_7$ | O | 96–99 |

Aus den erfindungsgemäßen Stoffen gemäß Beispielen 1 und (3) bis (12) werden durch Umsetzung mit Chloracetylchlorid bzw. Bromacetylchlorid die in der nachstehenden Tabelle 2 formelmäßig aufgeführten Verbindungen erhalten.

TABELLE 2

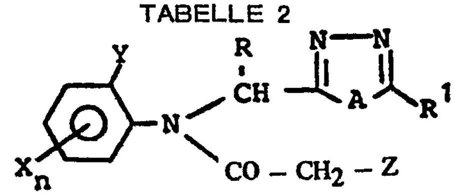

| Bsp.-Nr. | R | $R^1$ | Y | $X_n$ | A | Z | Schmelzpunkt (°C) |
|---|---|---|---|---|---|---|---|
| 3 a | H | $CH_3$ | $C_2H_5$ | 6-$C_2H_5$ | O | Cl | 79–82 |
| 4 a | H | $CH_3$ | $CH_3$ | 6-$CH_3$ | O | Cl | 91–93 |
| 5 a | H | $CH_3$ | $C(CH_3)_3$ | – | O | Cl | 102–04 |
| 6 a | H | $-S-CH_2-CH=CH_2$ | $C_2H_5$ | 6-$C_2H_5$ | 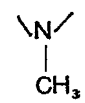 | Cl | |
| 7 a | H | 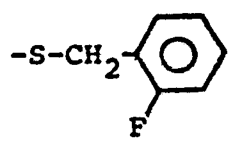 | $CH_3$ | 6-$C_2H_5$ | 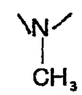 | Cl | 115–20 |
| 8 a | H | $C_2H_5$ | $CH_3$ | 6-$C_2H_5$ | O | Cl | 57–59 |
| 9 a | H | $C_2H_5$ | $C_2H_5$ | 6-$C_2H_5$ | O | Cl | 43–47 |
| 10 a | H | i-$C_3H_7$ | $CH_3$ | 6-$C_2H_5$ | O | Cl | zähfl. Öl |
| 11 a | H | $CH_3$ | $CH_3$ | 3-$CH_3$ | 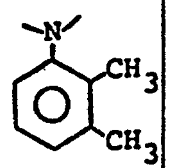 | Cl | glasartig erstarrt |
| 12 a | H | $CH_3$ | $C_2H_5$ | 6-$C_2H_5$ | O | Br | 80 |
| 13 a | H | $CH_3$ | $CH_3$ | 6-$C_2H_5$ | O | Br | 92–94 |
| 14 a | H | $CH_3$ | i-$C_3H_7$ | 6-i-$C_3H_7$ | O | Cl | 135–137 |

**Patentansprüche**

1. N-Azolylalkyl-aniline der Formel

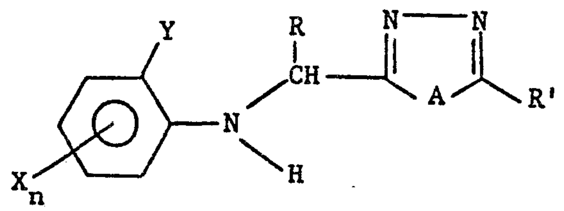

(I)

in welcher

A für Sauerstoff, Schwefel oder die Gruppierung $NR^2$ steht,

R für Wasserstoff oder Alkyl steht,

$R^1$ für Wasserstoff, Alkyl, Halogenalkyl, Alkenyl, Alkinyl, Cycloalkyl, Halogen, gegebenenfalls substituiertes Aryl und Aralkyl oder die Gruppierungen $—OR^3$, $—SR^3$ und $NR^2R^3$ steht,

$R^2$ für Wasserstoff, Alkyl, oder gegebenenfalls substituiertes Aryl steht,

$R^3$ für Wasserstoff, Alkyl, Halogenalkyl, Alkenyl, Alkinyl, Cycloalkyl oder gegebenenfalls substituiertes Aralkyl steht,

X für Alkyl steht,

Y für Alkyl oder Halogen steht und

n für die Zahlen 0, 1 oder 2 steht.

2. Verfahren zur Herstellung von N-Azolylalkyl-anilinen, dadurch gekennzeichnet, daß man

a) Aniline der Formel

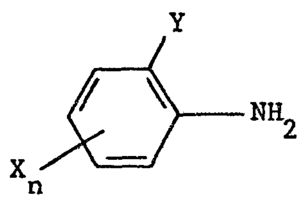

(II)

in welcher

X, Y und n die oben angegebene Bedeutung haben,

mit Azol-Derivaten der Formel

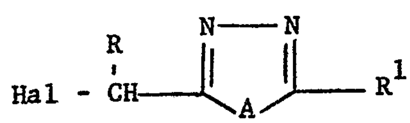

(III)

in welcher

A, R und $R^1$ die oben angegebene Bedeutung haben und

Hal für Chlor oder Brom steht,

in Gegenwart eines Säurebinders und in Gegenwart eines Verdünnungsmittels umsetzt, oder

b) Hydrazin-Derivate der Formel

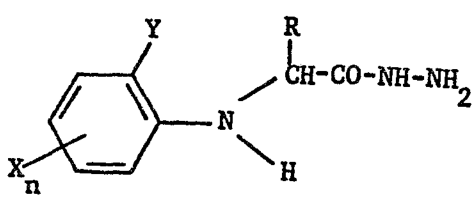

(IV)

in welcher

R, X, Y und n die oben angegebene Bedeutung haben,

mit Isocyanaten bzw. Senfölen der Formel

$$R^2—N{=}C{=}B \qquad (V)$$

in welcher
B für Sauerstoff oder Schwefel steht und
$R^2$ die oben angegebene Bedeutung hat,
in Gegenwart eines Verdünnungsmittels umsetzt, die dabei entstehenden Ver--bindungen der Formel

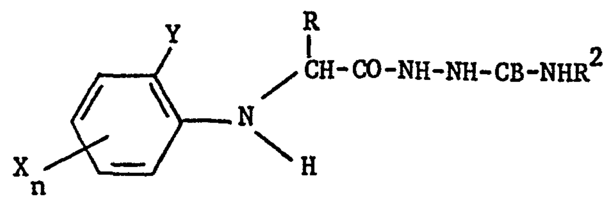

(VI)

in welcher
B, R, $R^2$, X, Y und n die oben angegebene Bedeutung haben,
in Gegenwart einer starken Base und in Gegenwart eines Verdünnungsmittels cyclisiert und die dabei enstehenden Triazolone bzw. Triazolthione der Formel

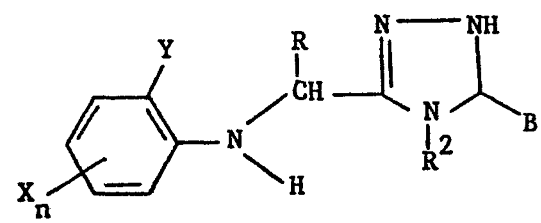

(VII)

in welcher
B, R, $R^2$, X, Y und n die oben angegebene Bedeutung haben,
mit Halogeniden der Formel

$$Hal—R^4 \qquad (VIII)$$

in welcher
Hal für Chlor oder Brom steht und
$R^4$ für die Reste des Substituenten $R^3$ steht, wobei Wasserstoff ausgenommen ist,
oder mit einem anderen Alkylierungsmittel als einem sochen der Formel (VIII) jeweils in Gegenwart einer starken Base und in Gegenwart eines Verdünnungsmittels sowie gegebenenfalls in Gegenwart eines Phasen-Transfer-Katalysators umsetzt.

**Revendications**

1. N-Azolylalkyl-anilines de formule:

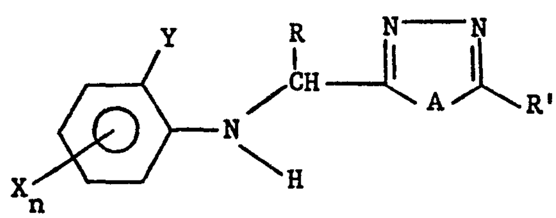

(I)

dans laquelle:
A représente l'oxygène, le soufre ou le groupement $NR^2$,
R représente l'hydrogène ou un groupe alkyle,
$R^1$ représente l'hydrogène, un groupe alkyle, halogénoalkyle, alcényle, alcynyle, cycloalkyle, un halogène, un groupe aryle ou aralkyle éventuellement substitué ou les groupements $—OR^3$, $—SR^3$ et $NR^2R^3$,
$R^2$ représente l'hydrogène, un groupe alkyle ou un groupe aryle éventuellement substitué,

$R^3$ représente l'hydrogène, un groupe alkyle, halogénoalkyle, alcényle, alcynyle, cycloalkyle ou un groupe aralkyle éventuellement substitué,

X représente un groupe alkyle,

Y représente un groupe alkyle ou un atome d'halogène, et

n représente l'un des nombres 0, 1 ou 2.

2. Procédé de préparation de N-azolylalkyl-anilines, caractérisé en ce que:

a) on fait réagir des anilines de formule:

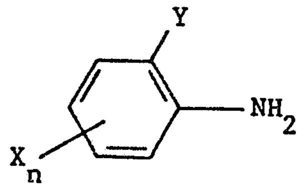

(II)

dans laquelle:

X, Y et n ont la signification indiquée ci-dessus, avec des dérivés d'azole de formule:

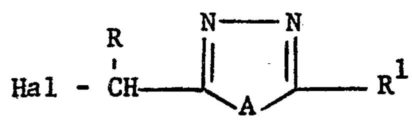

(III)

dans laquelle:

A, R et $R^1$ ont la signification indiquée ci-dessus et Hal représente le chlore ou le brome, en présence d'un accepteur d'acide et d'un diluant, ou bien

b) on fait réagir des dérivés d'hydrazine de formule:

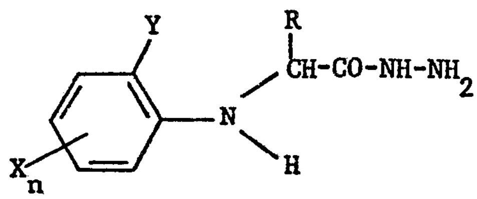

(IV)

dans laquelle:

R, X, Y et n ont la signification indiquée ci-dessus, avec des isocyanates ou des sénévols de formule:

$$R^2—N=C=B \qquad (V)$$

dans laquelle:

B représente l'oxygène ou le soufre, et

$R^2$ a la signification indiquée ci-dessus, en présence d'un diluant, ce qui donne des composés de formule:

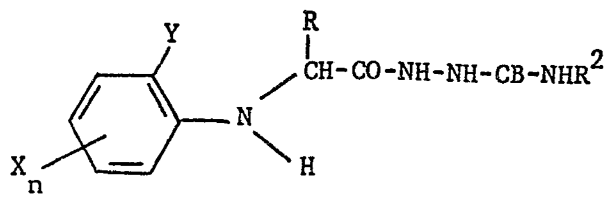

(VI)

dans laquelle:

B, R, $R^2$, X, Y et n ont la signification indiquée ci-dessus, qu'on cyclise en présence d'une base forte et d'un diliant, formant ainsi des triazolones ou triazolthiones, respectivement, de formule:

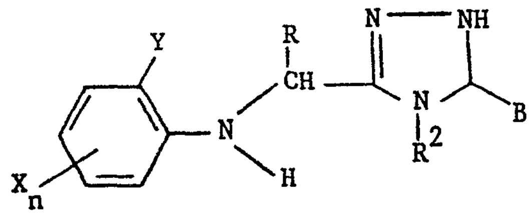

(VII)

dans laquelle:

B, R, $R^2$, X, Y et n ont la signification indiquée ci-dessus, qu'on fait réagir avec des halogénures de formule:

$$Hal—R^4 \qquad (VIII)$$

dans laquelle:

Hal représente le chlore ou le brome, et

$R^4$ représente les restes du substituant $R^3$, à l'exclusion de l'hydrogène, ou avec un agent alkylant autre qu'un composé de formule VIII, dans tous les cas en présence d'une base forte et d'un diluant et, le cas échéant, en présence d'un catalyseur de transfert de phase.

**Claims**

1. N-Azolylalkyl-anilines of the formula

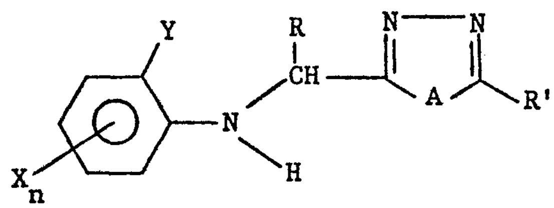

(I)

in which

A represents oxygen, sulphur or the grouping $>NR^2$,

R represents hydrogen or alkyl,

$R^1$ represents hydrogen, alkyl, halogenoalkyl, alkenyl, alkynyl, cycloalkyl, halogen, optionally substituted aryl, optionally substituted aralkyl or the grouping $—OR^3$, $—SR^3$ or $—NR^2R^3$,

$R^2$ represents hydrogen, alkyl or optionally substituted aryl,

$R_3$ represents hydrogen, alkyl, halogenoalkyl, alkenyl, alkynyl, cycloalkyl or optionally substituted aralkyl,

X represents alkyl,

Y represents alkyl or halogen and

*n* represents the number 0, 1 or 2.

2. A process for the preparation of N-azolylalkylanilines, characterised in that

(a) anilines of the formula

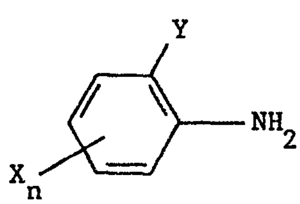

(II),

in which

X, Y and *n* have the meanings stated above,

are reacted with azole derivatives of the formula

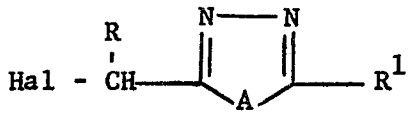

(III),

in which

A, R and $R^1$ have the meanings stated above and

Hal represents chlorine or bromine,

in the presence of an acid-binding agent and in the presence of a diluent, or

(b) hydrazine derivatives of the formula

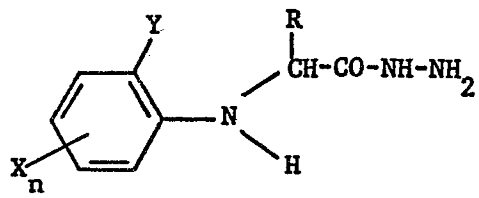

(IV),

in which

R, X, Y and *n* have the meanings stated above,

are reacted with isocyanates or isothiocyanates of the formula

$$R^2—N=C=B \quad (V),$$

in which

B represents oxygen or sulphur and

$R^2$ has the meaning stated above,

in the presence of a diluent, the compounds thereby formed, of the formula

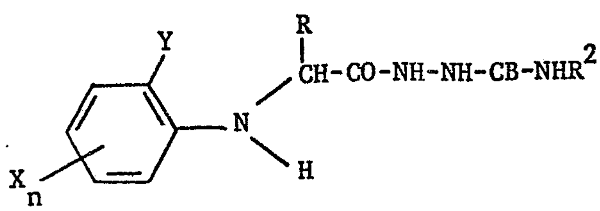

(VI),

in which

B, R, $R^2$, X, Y and *n* have the meanings stated above, are cyclised in the presence of a strong base and in the presence of a diluent, and the triazolones or triazolethiones thereby formed, of the formula

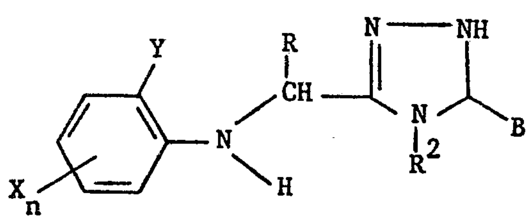

(VII),

in which

B, R, $R^2$, X, Y and n have the meanings stated above,

are reacted with halides of the formula

$$Hal—R^4 \quad (VIII)$$

in which

Hal represents chlorine or bromine and

$R^4$ represents the radicals of the substituent $R^3$, with the exception of hydrogen,

or with an alkylating agent other than one of the formula (VIII), in each case in the presence of a strong base and in the presence of a diluent as well as optionally in the presence of a phase transfer catalyst.